## Europäisches Patentamt
## European Patent Office
## Office européen des brevets

(19)

(11) Veröffentlichungsnummer: **0 001 127**
**B1**

(12) **EUROPÄISCHE PATENTSCHRIFT**

(45) Veröffentlichungstag der Patentschrift: 25.11.81

(21) Anmeldenummer: 78100874.3

(22) Anmeldetag: 13.09.78

(51) Int. Cl.³: **A 61 B 5/10, A 61 B 9/00, G 01 N 3/32**

(54) **Aus einem Geber für mechanische Schwingungen veränderbarer Frequenz und einem Schwingungsaufnehmer bestehende Einrichtung zur Bestimmung der mechanischen Eigenfrequenz von BlutgefäBen oder Sehnen im Körper.**

(30) Priorität: 14.09.77 DE 2741338

(43) Veröffentlichungstag der Anmeldung:
21.03.79 Patentblatt 79/6

(45) Bekanntmachung des Hinweises auf die Patenterteilung:
25.11.81 Patentblatt 81/47

(84) Benannte Vertragsstaaten:
BE CH FR GB NL SE

(56) Entgegenhaltungen:
DE - A - 2 101 960
FR - A - 735 152
US - A - 3 682 161
US - A - 3 782 365
US - A - 4 058 115

MEDICAL AND BIOLOGICAL ENGINEERING,
Band 14, Nr. 3, Mai 1976,
Steverage (GB)
G.A. THOMPSON, D. ORNE, D.R. YOUNG, "In vivo determination of mechanical properties of the human ulna by means of mechanical impedance tests: Experimental results and improved mathematical model", Seiten 253—262

(73) Patentinhaber: industrie-automation GmbH & Co.
Schlosswolfsbrunnenweg 35
D-6900 Heidelberg (DE)

(72) Erfinder: Hubmann, Max, Dr. med.
Rathsbergerstrasse 24
D-8520 Erlangen (DE)
Erfinder: Lang, Erich, Prof. Dr. med.
Am Veilchenberg 8
D-8521 Spardorf (DE)
Erfinder: Menke, Franz
Am Mühlrain 34
D-6903 Neckargemünd (DE)

(74) Vertreter: Dreyer, Karl-August
Pupinweg 25
D-6100 Darmstadt (DE)

Aus einem Geber für mechanische Schwingungen veränderbarer Frequenz und einem Schwingungsaufnehmer bestehende Einrichtung zur Bestimmung der mechanischen Eigenfrequenz von Blutgefäßen oder Sehnen im Körper.

Die Erfindung betrifft eine aus einem Geber für mechanische Schwingungen veränderbarer Frequenz und einem Schwingungsaufnemer bestehende Einrichtung zur Bestimmung der mechanischen Eigenfrequenz von Blutgefäßen oder Sehnen in Körpern, wobei diese als Koppelglieder zwischen dem Geber und dem Schwingungsaufnehmer wirken.

Derartige Einrichtungen dienen diagnostischen Zwecken, insbesondere dazu, Abweichungen von der normalen Eigenfrequenz oder deren zeitliche Veränderung von Organen, z.B. der Arteria radialis, zu bestimmen und auf diese Weise altersbedingte oder krankhafte Veränderungen zu erkennen.

Durch die Dissertation von Dr. Max Hubmann, Erlangen, 1973 sind Einrichtungen zur Bestimmung von solchen Eigenfrequenzen bekannt, bei denen über dem Körperteil, in dem sich das Organ befindet, dessen Eigenfrequenz gemessen werden soll, ein Geber für mechanische Schwingungen angeordnet ist, dessen in der Frequenz varänderbare Schwingungen auf den betreffenden Körperteil mechanisch übertragen werden und gesondert davon ein Schwingungsaufnehmer angeordnet ist. Maxima von dessen Ausgangsspannung bei bestimmten Frequenzen zeigen die Eigenfrequenz des Kopplungsgliedes zwischen Geber und Schwingungsaufnehmer an.

Diese bekannten Einrichtungen haben den Nachteil, daß ihre Einstellung sehr umständlich und die Erzielung reproduzierbarer Meßergebnisse sehr erschwert ist. Das wirkt sich besonders dann nachteilig aus, wenn durch Messungen in mehr oder weniger großen Zeitabständen die zeitliche Veränderung, z.B. durch Alterung, verfolgt werden soll.

Der Erfindung liegt die Aufgabe zugrunde, eine Einrichtung zu schaffen, die einfach einzustellen ist, unterschiedlichen Gegebenheiten leicht anzupassen ist und die dabei gut reproduzierbare Ergebnisse liefert.

Erfindungsgemäß wird diese Aufgabe bei der aus einem Geber für mechanische Schwingungen veränderbarer Frequenz und einem Schwingungsaufnehmer bestehende Einrichtung dadurch gelöst, daß die Übertragungsmittel vom Geber zum zum Körper und von ihm zum Schwingungsaufnehmer innerhalb eines Stützrohres untergebracht sind, dessen eines Ende starr mit dem Gestell, in dem der Geber und der Schwingungsaufnehmer gegenseitig entkoppelt, gegebenenfalls auch dem Schwingungsaufnehmer nachgeschaltete Verstärker oder dgl. angebracht sind, verbunden ist und dessen anderes Ende auf den Körper aufsetzbar ist.

Um die Reproduzierbarkeit der Messungen zu verbessern, werden zweckmäßig Mittel zur Einstellung des Aufsetzdrucks des Stützrohres vorgesehen.

In der Zeichnung sind Ausführungsbeispiele der erfindungsgemäßen Einrichtung dargestellt. Es zeigen

Fig. 1 einen Schnitt durch das Stützrohr und das Gestell,

Fig. 2 eine Vergrößerung eines Teils der Fig. 1,

Fig. 3 die Vorderansicht der Einrichtung für das Aufsetzen des Stützrohres auf den Körper von oben bzw. von unten,

Fig. 4 die Draufsicht einer Anordnung der Einrichtung für das seitliche Ansetzen an den Körper.

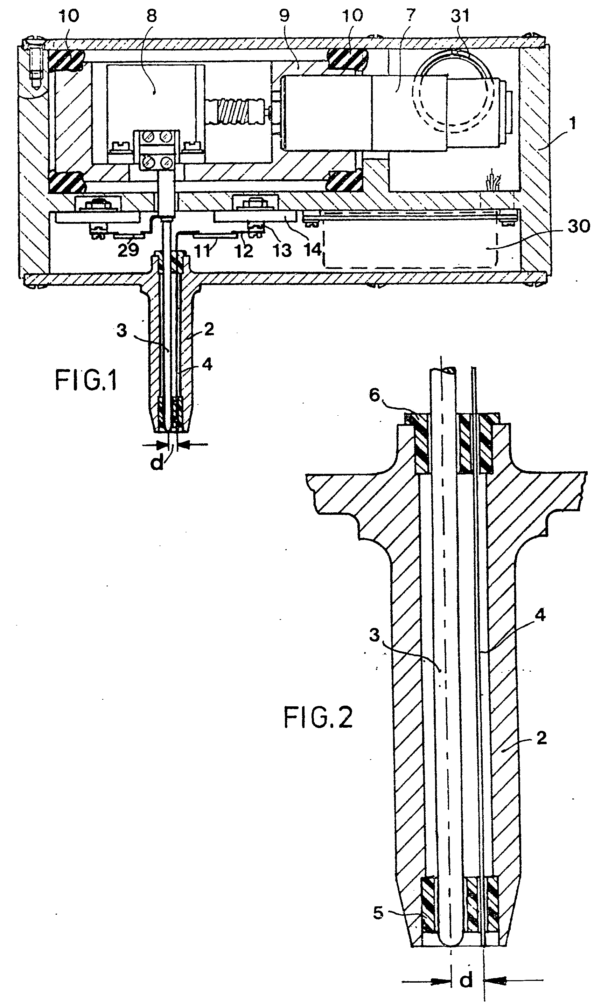

Der in Fig. 1 dargestellte Schnitt zeigt das Gestell 1, mit dem das Stützrohr 2 starr verbunden ist. In seinem Inneren sind der Stößel 3 und der Abtastfühler 4 achsparallel angeordnet. Wie Fig. 2, in der das Stützrohr gegenüber dem Maßstab der Fig. 1 vergrößert dargestellt ist, verdeutlicht, werden der Stößel 3 und der Abtastfühler 4 mittels der Führungsstücke 5 und 6 in dem stets gleichbleibenden Abstand d gehalten.

Sie bestehen zweckmäßig aus einem Material mit guten Gleiteigenschaften z.B. einem geeigneten Kunststoff. Auf diese Weise wird vermieden, daß die axialen Schwingungen des Stößels 3 durch Reibungskräfte auf die Führungsstücke 5 und 6 und von diesen auf den Abtastfühler 4 übertragen werden.

Der Geber für die mechanischen Schwingungen besteht aus dem in seiner Drehzahl regelbaren Elektromotor 7 und einer mit ihm gekuppelten Einrichtung 8, z.B. eines Kurbeltriebs oder einer Exzenteranordnung, mit deren Hilfe die Drehbewegung des Elektromotors 7 in eine Hin- und Herbewegung verwandelt und auf den Stößel 3 übertragen wird.

Der aus dem Elektromotor 7 und der Einrichtung 8 zur Umwandlung der Drehbewegung in eine Hin- und Herbewegung bestehende Geber für die mechanischen Schwingungen veränderbarer Frequenz sind in einem Rahmen 9 untergebracht. Er wird von federnden Klötzen 10 gehalten derart im Gestell 1 gelagert, daß keine Schwingungen des Gebers auf das Gestell und von diesem auf den ebenfalls an dem Gestell befestigten Schwingungsaufnehmer 11 einwirken können.

Dieser besteht in dem Ausführungsbeispiel aus einem piezoelektrisch arbeitendem mechanisch-elektrischen Wandler. Er ist über eine Feder 12 elektrisch leitend mit dem Anschlußbolzen 13 verbunden, der mittels des Isolierstückes 14 an dem Gestell 1 befestigt ist. Andererseits ist der Schwingungsaufnehmer 11 mit dem Abtastfühler 4 verbunden, der die mit seinem anderen Ende abgetasteten mechanischen Schwingungen an den Schwingungsaufnehmer weitergibt.

Die federnde Aufhängung schützt den Schwingungsaufnehmer vor Zerstörung bei unsachgemäßer Belastung des Abtastfühlers 4. Der Stößel 3 ist ebenfalls mit einem Schwingungsaufnehmer 29 verbunden. Seine Ausgangsspannung, die der Schwingung des Stößels 3 entspricht, stellt den Geber für die Frequenz der mechanischen Schwingungen dar. Die Ausgangsspannungen beider Schwingungsaufnehmer werden in der ebenfalls im Gestell 1 untergebrachten Elektronik 30 gelegt, deren Ausgangssignale an die Mehrfachsteckverbindung 31 geführt sind, von wo aus sie beispielsweise einem 2-Koordinatenschreiber zugeführt werden. Die Betriebsspannungen sowohl für die Elektronik 30 als auch für den Elektromotor 7 werden diesen über die Mehrfachsteckverbindung zugeleitet.

Werden die Schwingungen des Stößels 3, wie das in Fig. dargestellte Beispiel veranschaulicht, durch Aufsetzen des Stützrohres 2 auf ein Körperteil 15 übertragen, so werden schwingfähige Organe, die sich im Bereich der vom Stößel 3 auf den Körperteil 15 übertragenen Schwingungen befinden, z.B. die Arterie 16, zu Schwingungen angeregt, die vom Abtastfühler 4 an der Oberfläche des Körperteils 15 abgetastet werden. Die Amplitude dieser Schwingungen erreicht ein Maximum, wenn die Frequenz des Gebers mit der Eigenfrequenz des schwingfähigen Organs übereinstimmt.

Dadurch, daß der Stößel 3 und der Abtastfühler 4 einen definierten und konstanten Abstand aufweisen, wird die Reproduzierbarkeit der Messungen gegenüber den bekannten Einrichtungen erheblich verbessert. Eine weitere Verbesserung wird gemäß einer Weiterbildung der Erfindung dadurch erreicht, daß Mittel zur Einstellung des Druckes vorgesehen sind, mit dem das Stützrohr auf den Körper aufgesetzt wird.

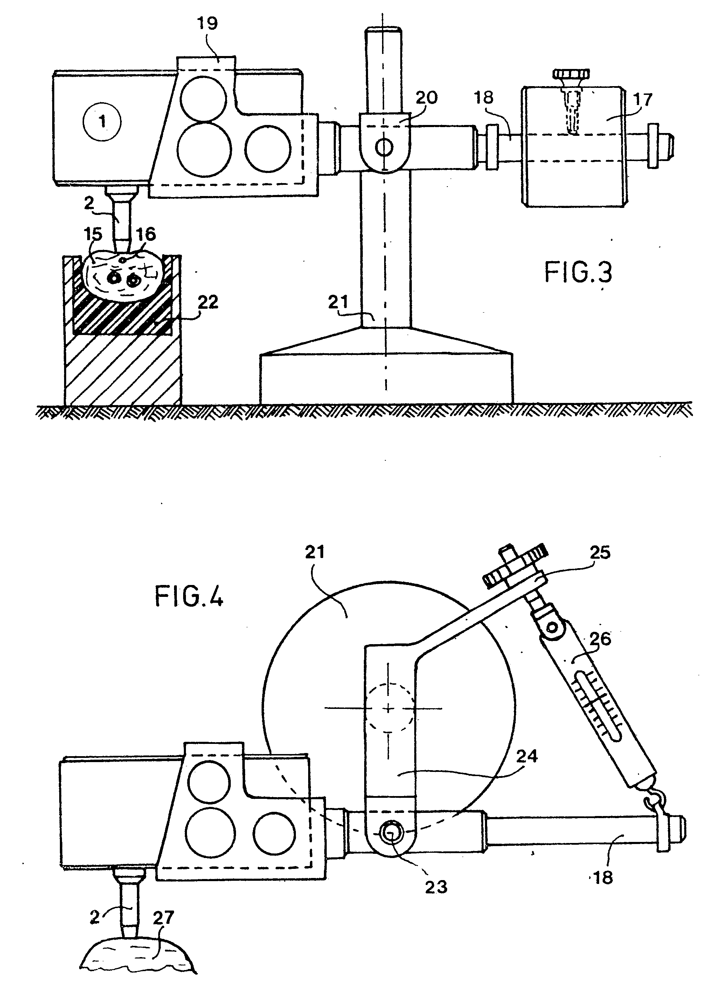

Wie Fig. 3 zeigt, kann die Einstellung des Aufsetzdruckes mittels eines Laufgewichts erfolgen, das auf dem einen Arm des Waagbalkens 18 verschiebbar ist, der an seinem anderen Arm eine Aufnahmevorrichtung 19 für das Gestell 1 trägt. Der Waagbalken 18 wird von einem Lager 20 getragen, das an einem Stativ befestigt ist. Nachdem das Laufgewicht zunächst derart eingestellt war, daß das Gewicht am anderen Arm bis auf den gewünschten Aufsetzdruck ausgeglichen wurde, wird die Höhe des Lagers 20 am Stativ 21 derart eingestellt, daß der Waagbalken 18 bei auf den Körperteil 15 aufgesetztem Stützrohr 2 horizontal liegt. Zur Erreichung einwandfreier Messungen, muß der Körper bzw. der Körperteil, in dem sich das Organ befindet, dessen Eigenfrequenz bestimmt werden soll, völlig ruhig gestellt werden. Das kann z.B. durch Lagerung der Körperteils in einem seiner Form individuell angepaßten Bett 22 erreicht werden.

Die in Fig. 3 dargestellte Anordnung ist nicht nur geeignet Messungen vorzunehmen, bei der das Stützrohr 2 von oben auf den Körper aufgesetzt wird, sondern sie erlaubt auch Messungen, bei denen das Stützrohr 2 von unten an den Körper angesetzt wird. Hierzu genügt es, das Gestell 1 so in die Aufnahmeeinrichtung 19 einzuführen, daß das Stützrohr 2 nach oben zeigt und das Laufgewicht 17 derart einzustellen, daß das Gewicht am anderen Arm des Waagbalkens 18 statt um den Betrag des gewünschten Aufsetzdruckes unterkompensiert, nunmehr um den gleichen Betrag überkompensiert wird.

Fig. 4 zeigt die Draufsicht einer Anordnung zur Einstellung des Aufsetzdruckes bei horizontal gerichtetem Stützrohr. Der Waagbalken 18 ist zu diesem Zwecke um die versikale Achse 23 drehbar in dem Haltestück 24 gelagert, das an dem Stativ 21 angebracht ist. Zwischen dem an dem Haltestück 24 befindlichen Einstellarm 23 und dem Waagbalken 18 ist die Federwaage 28 gespannt, mit der der Ansetzdruck des Stützrohres 2 an den Körper 27 gemessen werden kann. Gegebenenfalls ist der Ansetzdruck mittels der Einstellmutter 28 auf den gewünschten Wert einzujustieren.

**Patentansprüche**

1. Aus einem Geber (7, 8, 9) für mechanische Schwingungen veränderbarer Frequenz und einem Schwingungsaufnehmer (11) bestehende Einrichtung zur Bestimmung der mechanischen Eigenfrequenz von Blutgefäßen (16) oder Sehnen in Körpern, wobei diese als Koppelglieder zwischen dem Geber und dem Schwingungsaufnehmer wirken, dadurch gekennzeichnet, daß die Übertragungsmittel (3) vom Geber (7, 8, 9) zum Körper (15) und von ihm zum Schwingungsaufnehmer (11) innerhalb eines Stützrohres (2) untergebracht sind, dessen eines Ende starr mit dem Gestell (1), in dem der Geber (7, 8, 9) und der Schwingungsaufnehmer gegenseitig entkoppelt, gegebenenfalls auch dahintergeschaltete Verstärker oder dgl. (30) angebracht sind, verbunden ist und dessen anderes Ende auf den Körper aufsetzbar ist.

2. Einrichtung nach Anspruch 1, dadurch gekennzeichnet, daß Mittel zur Einstellung des Druckes vorgesehen sind, mit dem das Stützrohr (2) auf den Körper (15 bzw. 27) aufgesetzt wird.

3. Einrichtung nach Anspruch 1 und 2, dadurch gekennzeichnet, daß das Gestell (1) an einem Waagbalken (18) aufgehängt ist und der Aufsetzdruck des Stützrohrohres (2) mittels eines Laufgewichts (17) auf dem Waagbalken (18) einstellbar ist.

4. Einrichtung nach Anspruch 1 bis 3, dadurch gekennzeichnet, daß der Geber aus einem stetig regelbaren Elektromotor (7) besteht, dessen Drehbewegung mittels einer Vorrichtung (8) in eine Hin- und Herbewegung umgesetzt und auf den Stößel (3) übertragen wird.

5. Einrichtung nach Anspruch 1 bis 4, da-

durch gekennzeichnet, daß der Schwingungs-aufnehmer (11) aus einem federnd in dem Ge-stell (1) eingespannten mechanisch-elek-trischen Wandler besteht, auf den die von dem Abtastfühler an der Oberfläche des Körpers (15) aufgenommenen Schwingungen übertragen werden.

6. Einrichtung nach Anspruch 1 bis 5, da-durch gekennzeichnet, daß die Ausgangsspan-nung des Schwingungsaufnehmers (11) mittels eines 2-Koordinatenschreibers als Funktion der Frequenz des Gebers (7, 8, 9) aufgezeichnet wird.

## Claims

1. A device, consisting of a transmitter (7, 8, 9) for mechanical vibrations of a variable frequency and of a vibration pick-up (11), for determining the natural frequency of blood vessels (16) or tendons in bodies, these organs acting as coupling elements between the transmitter and vibration pick-up, characterised in that the transmission means (3) from the transmitter (7, 8, 9) to the body (15) and from the body to the vibration pick-up (11) are accommodated inside a support tube (2) one end of which is rigidly connected to the framework (1) in which the transmitter (7, 8, 9) and the vibration pick-up and, if necessary, also subsequent amplifiers or similar devices (30) are mounted in a mutually decoupled manner and the other end of which can be placed on the body.

2. A device according to Claim 1, characterised in that means are provided for adjusting the pressure with which the support tube (2) is applied to the body (15 or 27, respectively).

3. A device according to Claim 1 and 2, characterized in that the framework (1) is suspended on a scale arm (18) and that the pressure of application of the support tube (2) can be adjusted by means of a moving weight (17) on the scale arm (18).

4. A device according to Claim 1 to 3, characterised in that the transmitter consists of an infinitely adjustable electric motor (7), the rotation movement of which is converted by means of a mechanism (8) into a reciprocal movement and transferred to the pen (3).

5. A device according to Claim 1 to 4, characterised in that the vibration pick-up (11) consists of a mechanical/electrical transducer which is elastically clamped in the framework (1) and to which the vibrations picked up by the sensor on the surface of the body (15) are transmitted.

6. A device according to Claim 1 to 5, characterised in that the output voltage of the vibration pick-up (11) is recorded by means of an X—Y recorder as a function of the frequency of the transmitter (7, 8, 9).

## Revendications

1. Dispositif constitué d'un générateur (7, 8, 9) d'oscillations mécaniques de fréquence variable et d'un capteur d'oscillations (11) et destiné à déterminer la fréquence mécanique propre de vaisseaux sanguins (16) ou de tendons dans des corps, ces organes servant d'éléments de couplage entre le générateur et le capteur d'oscillations, caractérisé en ce que, les moyens de transmission (3) du générateur (7, 8, 9) au corps (15) et du corps au capteur d'oscillations (11) sont logés dans un tube de support (2) dont une extrémité est reliée rigidement au bâti (1) dans lequel sont montés, désaccouplés l'un de l'autre, le générateur (7, 8, 9) et le capteur d'oscillations, ainsi que, le cas échéant, l'amplificateur ou le dispositif analogue (30) connecté en aval du capteur, et dont l'autre extrémité peut être posée sur le corps.

2. Dispositif suivant la revendication 1, caractérisé en ce que, des moyens sont prévus pour régler la pression sous laquelle le tube de support (2) est posé sur le corps (15 ou 27).

3. Dispositif suivant les revendications 1 et 2, caractérisé en ce que, le bâti (1) est suspendu à un fléau (18) et la pression d'appui du tube de support (2) peut être réglée au moyen d'un poids mobile (17) sur le fléau (18).

4. Dispositif suivant les revendications 1 à 3, caractérisé en ce que, le générateur est constitué d'un moteur électrique réglable de manière continue (7) dont le mouvement de rotation est converti au moyen d'un dispositif (8) en un mouvement alternatif et est transmis au poussoir (3).

5. Dispositif suivant les revendications 1 à 4, caractérisé en ce que, le capteur d'oscillations (11) est constitué d'un transducteur mécanique-électrique monté élastiquement dans le bâti (1), auquel sont transmises les oscillations captées par la sonde palpeuse sur la surface du corps (15).

6. Dispositif suivant les revendications 1 à 5, caractérisé en ce que, la tension de sortie du capteur d'oscillation (11) est enregistrée au moyen d'un enregistreur électronique à deux coordonnées en fonction de la fréquence du générateur (7, 8, 9).

FIG.1

FIG.2

FIG.3

FIG.4